(19) Europäisches Patentamt
European Patent Office
Office européen des brevets

(11) Veröffentlichungsnummer: **0 032 658**
**B1**

(12) # EUROPÄISCHE PATENTSCHRIFT

(45) Veröffentlichungstag der Patentschrift: **21.03.84**

(51) Int. Cl.³: **A 61 K 7/00**, A 61 K 9/08

(21) Anmeldenummer: **81100020.7**

(22) Anmeldetag: **05.01.81**

(54) **Verwendung von Di-tert.-alkylphenolethoxylaten als Lösungsvermittler für kosmetische und pharmazeutische Präparate.**

(30) Priorität: **16.01.80 DE 3001302**

(43) Veröffentlichungstag der Anmeldung: **29.07.81 Patentblatt 81/30**

(45) Bekanntmachung des Hinweises auf die Patenterteilung: **21.03.84 Patentblatt 84/12**

(84) Benannte Vertragsstaaten: **AT BE CH DE FR GB IT LI NL SE**

(56) Entgegenhaltungen:
**DE-B- 2 255 314**
**DE-C- 865 510**
**NL-A- 6 610 056**
**US-A- 4 020 183**
**US-A- 4 199 575**
**TENSIDE, DETERGENTS, Band 11, Heft 3, Mai/Juni 1974 MÜNCHEN (DE) PENZEL u.a.: Charakterisierung einiger nichtionogener Emulgatoren: Einige kritische Anmerkungen zum HLB-System", Seiten 129-136**

(73) Patentinhaber: **BASF Aktiengesellschaft**
**Carl-Bosch-Strasse 38**
**D-6700 Ludwigshafen (DE)**

(72) Erfinder: **Seib, Karl, Dr.**
**Kriemhildstrasse 36**
**D-6940 Weinheim (DE)**
Erfinder: **Krapf, Heinz, Dr.**
**In der Schleit**
**D-6718 Gruenstadt (DE)**
Erfinder: **Oppenlaender, Knut, Dr. Chem.**
**Otto-Dill-Strasse 23**
**D-6700 Ludwigshafen (DE)**

EP 0 032 658 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verwendung von Di-tert.-alkylphenolethoxylaten als Lösungsvermittler für kosmetische und pharmazeutische Präparate

Die Erfindung betrifft eine neue Verwendungsmöglichkeit für äthoxylierte Alkylphenole, welche 2 tertiäre Alkylgruppen gebunden enthalten, als Lösungsvermittler in der kosmetischen und pharmazeutischen Industrie.

Für die Herstellung von klaren, homogenen kosmetischen oder pharmazeutischen wäßrigen oder wäßrig/alkoholischen Zubereitungen (z. B. Kölnisch Wasser, Rasierwasser, Haarfestiger, Vitamin-A-Zubereitungen, Steringlycosid-Zubereitungen) hat die Lösungsvermittlung (Solubilisierung) eine große Bedeutung. Hierzu setzt man Lösungsvermittler ein. Es handelt sich hierbei um Stoffe, welche in Wasser unlösliche, in mit Wasser nicht mischbaren Lösungsmitteln jedoch lösliche Stoffe, zu denen auch Zusatzstoffe zu den obenerwähnten Kosmetika und Pharmazeutika zählen, in Wasser oder Wasser-Alkohol-Gemischen in Lösung bringen können.

Als gute Lösungsvermittler werden z. B. in der DE-PS-15 92 996 Äthoxylate des hydrierten Ricinusöls beschrieben.

Die Verarbeitung wird i. a. in der Weise durchgeführt, daß man, um Gelbildungen, die vor allem beim Verdünnen der Zubereitungen auftreten können, zu vermeiden, das Präparat und den Lösungsvermittler bei Temperaturen um 50 °C innig vermischt und die Mischung mit warmem oder kaltem Wasser unter Rühren verdünnt.

Nachteilig ist diese bislang notwendige Temperaturbedingung bei der Verarbeitung von empfindlichen Präparaten (Temperaturemfindliche, z. B. Mehrfachbindungen aufweisende Präparate), die sich bei diesen Temperaturen häufig schon zersetzen oder umlagern (Terpene, Steroide, Carotinoide), was ein Arbeiten bei Raumtemperatur und darunter erforderlich macht.

Bei dieser Temperatur traten aber bisher häufig die erwähnten Gelbildungen (Hydratation der Lösungsvermittler) auf, welche die Verarbeitung sehr erschwerten. Bei einigen wenigen Produkten konnte dieser Nachteil nur dadurch vermieden werden, daß man wesentlich größere Mengen an Lösungsvermittler einsetzte.

Das Ziel der Erfindung bestand in der Auffindung von geeigneten Substanzen, mit denen ein einwandfreies Arbeiten auch bei Raumtemperatur und mit weniger Lösungsvermittler eine Lösungsvermittlung in einem rein wäßrigen System ohne Schwierigkeiten möglich sind. Es wurde überraschenderweise gefunden, daß mit gewisse Alkylgruppen mit tertiären C-Atomen tragenden Phenolethoxylaten, wie sie gemäß dem Anspruch definiert sind, dieses Ziel erreicht werden kann.

Diese Verbindungen zeigen in gewisser Menge eingesetzt auch bei Raumtemperatur die erwähnten Hydratationserscheinungen nicht mehr und ermöglichen nunmehr auch die Solubilisierung empfindlicher Präparate. Die erfindungsgemäß einzusetzenden Phenolderivate tragen am aromatischen Kern zwei tertiäre Alkylgruppen sowie gegebenenfalls eine weitere $C_1$- bis $C_3$-Alkylgruppe, wobei der Methylrest bevorzugt ist. Die tert. Alkylgruppen haben 4 bis 6 C-Atome und es seien tert.-Butyl-, tert.-Amyl und die verschiedenen isomeren tert. Hexylgruppen beispielhaft genannt.

Bevorzugte Verbindungen basieren auf 2,4-Di-tert.-bytylphenol und 2,6-Di-tert.-butyl-p-kresol.

Die Phenole sind mit 10 bis 100, vorzugsweise 10 bis 40 Oxäthylgruppen ethoxyliert. Von besonderem Interesse sind 12- bis 16-fach ethoxylierte Phenole.

Um gute Ergebnisse zu erzielen, setzt man — bezogen auf 1 Gew.-Teil zu solubilisierender Substanz 0,5 bis 8, vorzugsweise 0,5 bis 5 Gew.-Teile Solubilisator zu. Substanzen, die erfindungsgemäß solubilisiert werden können, sind beispielsweise kosmetische Duftstoffe, wie Linalool, Terpengemische, Isophytol, Parfümöl-Kompositionen, Fett- und Ölkomponenten, fettlösliche Pharmazeutika, wie Vitamin-A-ester, -alkohol, Vitamin E, fettlösliche Antibiotika z. B. der Tetracyclin-Reihe, Steringlycoside, wie Digitalis-Glycoside oder Steroidhormone.

Kosmetische Präparationen, bei deren Herstellung die erfindungsgemäß einzusetzenden Lösungsvermittler Verwendung finden, sind z. B. Rasierwasser, Parfüms, Eau de Toilets, Eau de Cologne, Haarshampoos, Schaumbäder oder Haarfestiger.

In der Pharmazie sind es vor allem Zubereitungen für Injektionen oder oral oder rectal zu verabreichende flüssige Präparate.

Die Anwendung der Phenolderivate ist denkbar einfach, indem man das Präparat mit dem Lösungsvermittler mischt und mit Wasser verdünnt, ohne daß hierbei eine Temperaturerhöhung notwendig ist.

Aus der folgenden Tabelle gehen die solubilisierenden Eigenschaften der untersuchten Lösungsvermittler hervor. Geprüft wurde in reinem Wasser. Die Zahlen geben die Minimalmenge an Lösungsvermittler wieder, die erforderlich ist, um bei 20 °C 1 g Riechstoff in 100 ml Wasser klar in Lösung zu brigen. Die hinter den Zahlen angegebenen Symbole zeigen an, ob und wie stark während der Solubilisierung Gelbildungen zu beobachten waren.

Die Symbole bedeuten :

++ = keine Gelbildung
\+ = leichte Gelbildung

– = Gelbildung
–– = starke Gelbildung

Getestet wurden folgende Substanzen:

1) Hydriertes Ricinusöl + 40 Ethylenoxid (EO)
2) dto + 60 EO
3) Nonylphenol + 10 EO
4) dto + 14 EO (Stand der Technik)
5) Polyoxäthylsorbitanmonolaurat
6) Polyoxäthylsorbitanmonooleat
7) $C_{12}$-$C_{16}$-Alkohol + 11 EO

8) 2,4-Di-tert.-butylphenol + 13 EO
9) dto + 12 EO
10) Di-tert.-butyl-p-kresol + 13 EO (erfindungsgemäß eingesetzte Substanzen)
11) dto + 15 EO
12) dto + 20 EO

Tabelle

| | Terpengemisch | | Isophytol | | Linalool | | Eau de Cologne Parfümöl | |
|---|---|---|---|---|---|---|---|---|
| Lösungsvermittler Nummer | | | | | | | | |
| 1 | 1,0 | ++ | 5,0 | - | 2,5 | - | 4,0 | - |
| 2 | 1,5 | + | 5,0 | -- | 2,5 | - | 4,0 | -- |
| 3 | 4,0 | + | 5,0 | - | 5,0 | - | 0,7 | + |
| 4 | 0,9 | ++ | 5,0 | -- | 1,5 | + | 4,0 | -- |
| 5 | 3,0 | ++ | 5,0 | + | 4,5 | + | 5,0 | + |
| 6 | 4,0 | - | 5,0 | -- | 1,5 | - | 2,5 | -- |
| 7 | 1,0 | ++ | 5,0 | - | 2,0 | + | 2,0 | - |
| 8 | 0,6 | ++ | 5,0 | + | 2,5 | ++ | 2,0 | + |
| 9 | 0,6 | ++ | 5,0 | + | 1,5 | ++ | 2,5 | + |
| 10 | 2,0 | ++ | 4,0 | ++ | 4,0 | ++ | 1,5 | + |
| 11 | 0,7 | ++ | 5,0 | + | 1,5 | ++ | 5,0 | + |
| 12 | 0,9 | ++ | 5,0 | + | 2,0 | + | 5,0 | + |

**Anspruch**

Verwendung von Verbindungen der Formel I

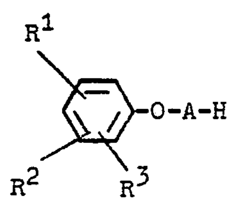

(I)

in der $R^1$ und $R^2$ gleich oder verschieden sind und für $C_4$- bis $C_6$-tert.-Alkylgruppen stehen, $R^3$ Wasserstoff oder eine $C_1$- bis $C_3$-Alkylgruppe und A den Rest eines Polyäthylenglykols mit 10 bis 100 Ethylenoxideinheiten bedeuten, als Lösungsvermittler für öllösliche Kosmetika und Pharmazeutika.

**Claim**

The use of compounds of the formula I

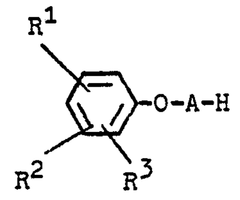

(I)

where $R^1$ and $R^2$ are identical or different and each denotes a tert.-alkyl of 4 to 6 carbon atoms, $R^3$ is hydrogen or alkyl of 1 to 3 carbon atoms, and A is the radical of a polyethylene glycol containing 10 to 100 ethylene oxide units, as solubilizers for oil-soluble cosmetics and pharmaceuticals.

**Revendication**

Utilisation, comme tiers solvant pour produits pharmaceutiques et cosmétiques solubles dans l'huile, de composés de formule I

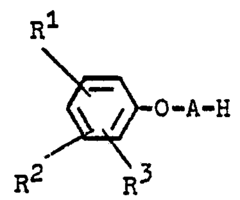

(I)

dans laquelle $R^1$ et $R^2$ sont identiques ou différents et représentent des groupes tert-alkyle en $C_4$ à $C_6$, $R^3$ représente l'hydrogène ou un groupe alkyle en $C_1$ à $C_3$ et A le reste d'un polyéthylèneglycol ayant 10 à 100 motifs éthylène-oxyde.